# EUROPEAN PATENT APPLICATION

(11) **EP 4 378 321 A1**
(43) Date of publication of application: **05.06.2024**
(21) Application number: 22849588.3
(22) Date of filing: 28.07.2022
(51) Int. Cl.: A23L 2/38, A23L 2/00, A23L 2/39, A23L 2/52, A23L 31/00, A23L 33/135, C12N 1/20

(54) **LACTOBACILLUS-CONTAINING BEVERAGE AND METHOD FOR PRODUCING SAME**

(30) Priority: 28.07.2021 JP 2021123383
(71) Applicant: KIRIN HOLDINGS KABUSHIKI KAISHA, Nakano-ku, Tokyo 164-0001 (JP)
(72) Inventor: HIROSE Yu, Tokyo 164-0001 (JP); SUGIHARA Yoshihiko, Tokyo 164-0001 (JP); KOKUBO Takeshi, Tokyo 164-0001 (JP); TSUJI Toshikazu, Tokyo 164-0001 (JP); MURAKAWA Hazuki, Tokyo 164-0001 (JP)
(74) Representative: Hoffmann Eitle
(86) International application number: PCT/JP2022/029123
(87) International publication number: WO 2023/008527

(57) **Abstract**

An object of the present invention is to provide a novel lactic acid bacterium-containing beverage and a method for producing the same, a novel lactic acid bacterium bulk powder and a method for producing the same, as well as a novel method for improving the water dispersibility of a lactic acid bacterium in a lactic acid bacterium-containing beverage. The present invention provides a lactic acid bacterium-containing beverage having a pH of 5.0 or higher at 25°C; and a method for producing a lactic acid bacterium-containing beverage, comprising the steps of: adjusting the pH of the beverage at 25°C to 5.0 or higher; and/or blending a lactic acid bacterium bulk powder obtained by heat-treating a lactic acid bacterium at a temperature of 90°C or higher. The present invention also provides a lactic acid bacterium bulk powder obtained by heat treatment at a temperature of 90°C or higher; and a method for producing the same. The present invention further provides a method for improving the water dispersibility of a lactic acid bacterium, comprising the steps of: adjusting the pH of a lactic acid bacterium-containing beverage at 25°C to 5.0 or higher; and/or blending a lactic acid bacterium bulk powder obtained by heat-treating a lactic acid bacterium at a temperature of 90°C or higher.

## Description

### CROSS-REFERENCE TO RELATED APPLICATION

The present application enjoys the benefit of priority from the prior Japanese Patent Application No. 2021-123383 filed on July 28, 2021, the entire disclosure of which is incorporated herein by reference.

### TECHNICAL FIELD

The present invention relates to a lactic acid bacterium-containing beverage and a method for producing the same. The present invention also relates to a lactic acid bacterium bulk powder and a method for producing the same, as well as a method for improving the water dispersibility of a lactic acid bacterium in a lactic acid bacterium-containing beverage.

### BACKGROUND ART

Normally, in a lactic acid bacterium-containing beverage, a lactic acid bacterium is not hydrated in an aqueous solution, but exists in a dispersed state. However, there is a problem that the water dispersibility of the lactic acid bacterium in the aqueous solution deteriorates depending on the composition of the beverage and the processed state of the lactic acid bacterium, resulting in sedimentation of the lactic acid bacterium due to aggregation thereof in the aqueous solution. As beverages improved in water dispersibility of a lactic acid bacterium, for example, those described in Patent Documents 1 and 2 have been known so far.

### Reference List

### Patent Documents

Patent Document 1: JP2016-214107 A
Patent Document 2: WO 2018/230585

### SUMMARY OF THE INVENTION

An object of the present invention is to provide a novel lactic acid bacterium-containing beverage and a method for producing the same. Another object of the present invention is to provide a novel lactic acid bacterium bulk powder and a method for producing the same, as well as a novel method for improving the water dispersibility of a lactic acid bacterium in a lactic acid bacterium-containing beverage.

The present inventors have found that, in a lactic acid bacterium-containing beverage, the pH of the beverage is adjusted to 5.0 or higher, thereby improving the water dispersibility of the lactic acid bacterium in the beverage. The present inventors have also found that, in a process for preparing a lactic acid bacterium bulk powder, the including of a lactic acid bacterium heat-treated at 90°C or higher in a beverage improves the water dispersibility of the lactic acid bacterium in the beverage and the retention of the water dispersibility of the lactic acid bacterium. The present invention is based on these findings.

According to the present invention, the following inventions are provided.
[1] A lactic acid bacterium-containing beverage having a pH of 5.0 or higher at 25°C.
[2] The beverage according to [1], which has a relative turbidity of 0.40 or more, wherein the "relative turbidity" is defined as a ratio of the turbidity (OD600) of the beverage when 20 hours have passed after start of still-standing to the turbidity (OD600) of the beverage at the start of the still-standing (0 hours) in a state where a lactic acid bacterium is dispersed in the beverage, and allowed to stand.
[3] The beverage according to [1] or [2], wherein the median diameter value of lactic acid bacterium particles, as measured by a laser diffraction particle size distribution measurement method, is 2 µm or less.
[4] The beverage according to any one of [1] to [3], wherein a heat-treated lactic acid bacterium bulk powder is used as a raw material.
[5] The beverage according to any one of [1] to [4], wherein the lactic acid bacterium is a dead bacterium.
[6] The beverage according to any one of [1] to [5], which is substantially free of an emulsifier and/or a thickener.
[7] The beverage according to any one of [1] to [6], wherein the number of lactic acid bacteria is 1 million or more/500 mL.
[8] The beverage according to any one of [1] to [7], which is a packaged beverage.
[9] The beverage according to any one of [1] to [8], wherein the lactic acid bacterium is a lactic acid coccus.
[10] The growth promoter according to [9], wherein the lactic acid coccus is a bacterium belonging to the genus *Lactococcus.*
[11] The beverage according to [10], wherein the bacterium belonging to the genus *Lactococcus* is a *Lactococcus lactis* subsp. *lactis* JCM5805 strain.
[12] A method for producing a lactic acid bacterium-containing beverage, comprising the steps of: (A) adjusting the pH of the beverage at 25 °C to 5.0 or higher, and/or (B) blending a lactic acid bacterium bulk powder obtained by heat-treating a lactic acid bacterium at a temperature of 90°C or higher.
[13] The method for producing a lactic acid bacterium-containing beverage according to [12], further comprising the step of (E) measuring the number of lactic acid bacterial cells in the lactic acid bacterium-containing beverage.
[14] A lactic acid bacterium bulk powder obtained by heat treatment at a temperature of 90°C or higher.
[15] The lactic acid bacterium bulk powder according to [14], wherein the ratio of one lactic acid bacterial cell to all bacterial cells is 80% or more.
[16] The lactic acid bacterium bulk powder according to [14] or [15], wherein the adhesive force of lactic acid bacterial cells in the lactic acid bacterium bulk powder is 0.83 or less relative to the adhesive force of lactic acid bacterial cells in the lactic acid bacterium bulk powder heat-treated at 80°C.
[17] A method for producing a lactic acid bacterium bulk powder, comprising the step of (D) heat-treating a lactic acid bacterium at a temperature of 90°C or higher.
[18] The method for producing a lactic acid bacterium bulk powder according to [17], wherein the lactic acid bacterium is killed in the step (D).
[19] A lactic acid bacterium bulk powder produced by the method for producing a lactic acid bacterium bulk powder according to [17] or [18].
[20] A method for improving the water dispersibility of a lactic acid bacterium in a lactic acid bacterium-containing beverage, comprising the steps of: (F) adjusting the pH of the beverage at 25°C to 5.0 or higher, and/or (G) blending a lactic acid bacterium bulk powder obtained by heat-treating a lactic acid bacterium at a temperature of 90°C or higher.
[21] The method for improving the water dispersibility of a lactic acid bacterium according to [20], for use in measuring the number of lactic acid bacterial cells contained in the lactic acid bacterium-containing beverage.

The present invention is advantageous in that the water dispersibility of a lactic acid bacterium in a lactic acid bacterium-containing beverage can be improved by adjusting the pH of the beverage to 5.0 or higher and/or including a lactic acid bacterium bulk powder heat-treated at 90°C or higher in the beverage, and thus that the number of lactic acid bacterial cells in the beverage can be measured conveniently and efficiently.

### BRIEF DESCRIPTION OF THE DRAWINGS

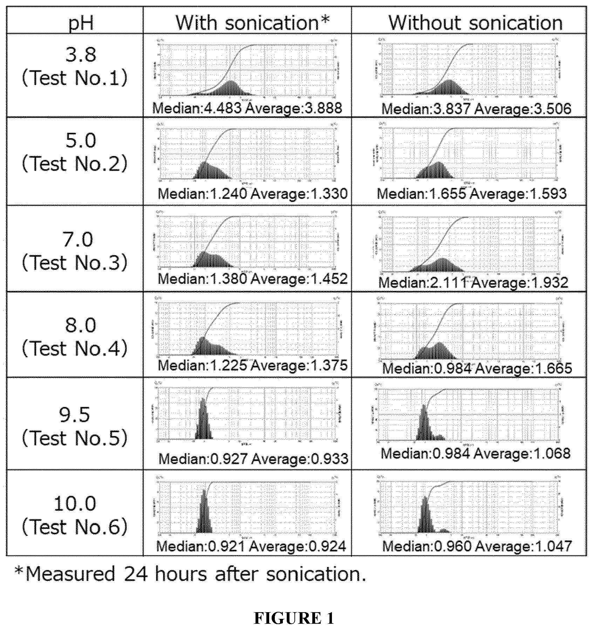

FIG. 1 shows particle size distributions of lactic acid bacterium particles in an aqueous solution adjusted to each pH, as well as their median diameter values and average values thereof (in each graph, the "Median" represents the median diameter value, and the "Average" represents the average value, both in µm). In each graph, the vertical axis indicates the frequency, and the horizontal axis indicates the particle size (in µm).

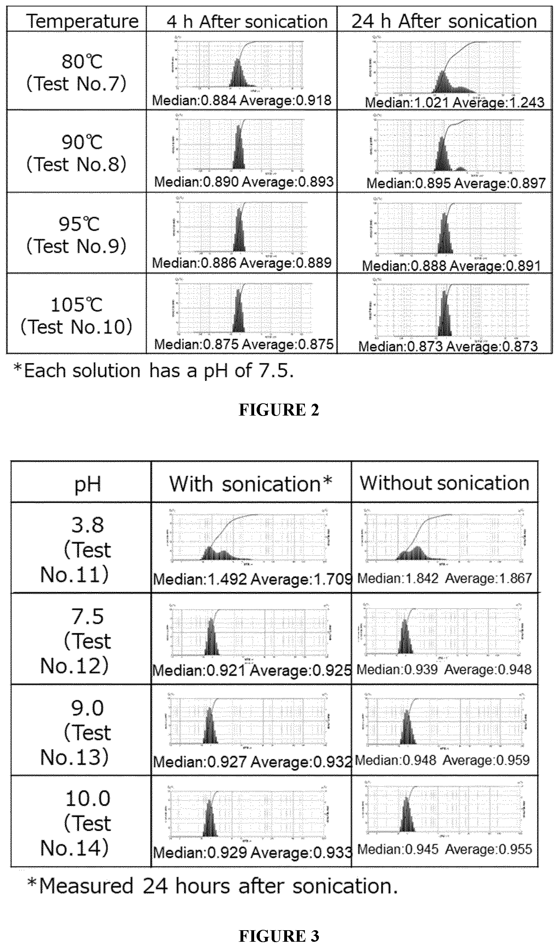

FIG. 2 shows particle size distributions of lactic acid bacterium particles in an aqueous solution containing a lactic acid bacterium bulk powder heat-treated at each temperature, as well as their median diameter values and average values thereof (in each graph, the "Median" represents the median diameter value, and the "Average" represents the average value, both in µm). In each graph, the vertical axis indicates the frequency, and the horizontal axis indicates the particle size (in µm).
FIG. 3 shows particle size distributions of lactic acid bacterium particles in an aqueous solution having each pH, the aqueous solution containing a lactic acid bacterium bulk powder heat-treated at 105°C, as well as their median diameter values and average values thereof (in each graph, the "Median" represents the median diameter value, and the "Average" represents the average value, both in µm). In each graph, the vertical axis indicates the frequency, and the horizontal axis indicates the particle size (in µm).

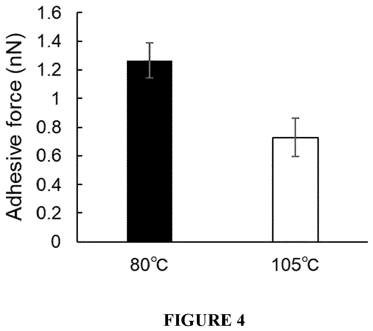

FIG. 4 shows the adhesive force of the lactic acid bacterial cell in a lactic acid bacterium bulk powder heat-treated at 80°C or 105°C.

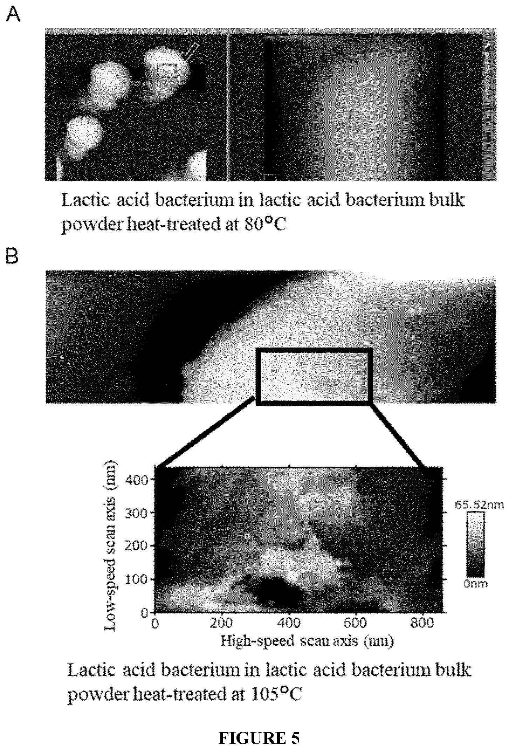

FIG. 5A shows an AFM image of the surface of the lactic acid bacterial cell in the lactic acid bacterium bulk powder heat-treated at 80°C. FIG. 5B shows an AFM image of the surface of the lactic acid bacterial cell in the lactic acid bacterium bulk powder heat-treated at 105°C.

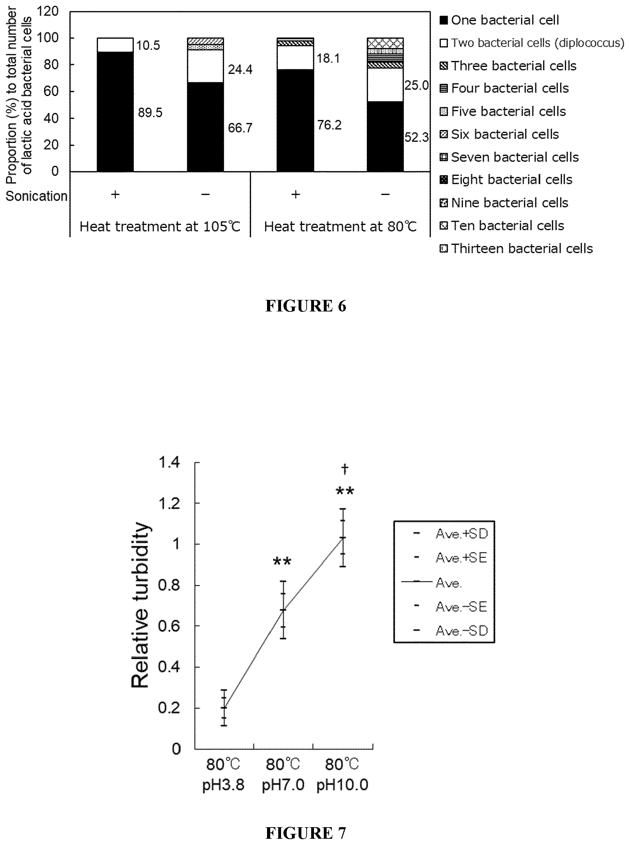

FIG. 6 shows the proportion of each lactic acid bacterial cell number to the total number of lactic acid bacterial cells in the aqueous solution containing the lactic acid bacterium bulk powder heat-treated at 80°C or 105°C. The symbols "+" and "-" indicate the presence or absence, respectively, of sonication.
FIG. 7 shows the relative turbidity when each lactic acid bacterium bulk powder heat-treated at 80°C is dispersed in PBS having a pH of 3 .8, 7.0, or 10.0. ** indicates a significant difference (p<0.01) relative to the numerical value at a pH of 3.8, and † indicates a significant difference (p<0.05) relative to the numerical value at a pH of 7.0. SD denotes standard deviation, and SE denotes standard error.

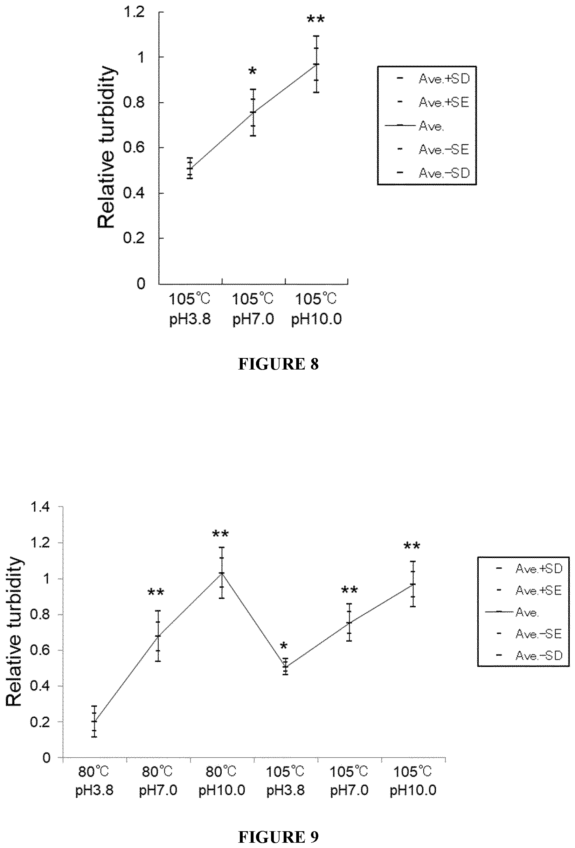

FIG. 8 shows the relative turbidity when each lactic acid bacterium bulk powder heat-treated at 105°C is dispersed in PBS having a pH of 3.8, 7.0, or 10.0. * indicates a significant difference (p<0.05) relative to the numerical value at a pH of 3.8, and ** indicates a significant difference (p<0.01) relative to the numerical value at a pH of 3.8. SD denotes standard deviation, and SE denotes standard error.
FIG. 9 shows the relative turbidity when each lactic acid bacterium bulk powder heat-treated at 80°C or 105°C is dispersed in PBS having a pH of 3.8, 7.0, or 10.0. * indicates a significant difference (p<0.05) relative to the numerical value at 80°C and a pH of 3 .8, and ** indicates a significant difference (p<0.01) relative to the numerical value at 80°C and a pH of 3.8. SD denotes standard deviation, and SE denotes standard error.

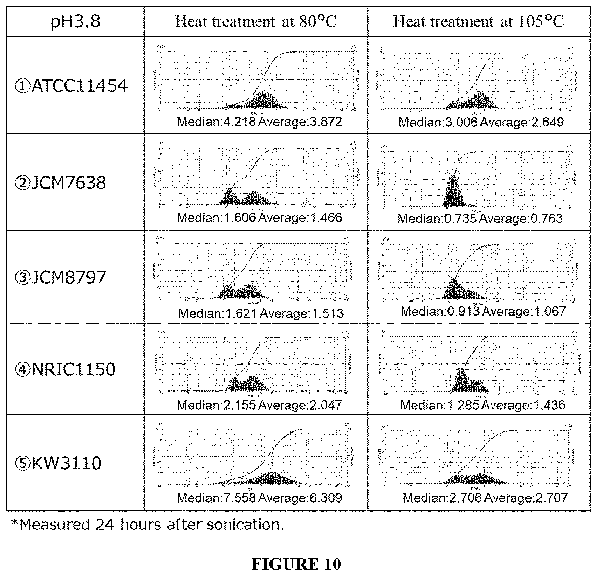

FIG. 10 shows particle size distributions of lactic acid bacterium particles in an aqueous solution containing each lactic acid bacterium bulk powder heat-treated at a pH of 3.8 and 80°C or 105°C, as well as their median diameter values and average values thereof (in each graph, the "Median" represents the median diameter value, and the "Average" represents the average value, both in µm). In each graph, the vertical axis indicates the frequency, and the horizontal axis indicates the particle size (in µm).

### DETAILED DESCRIPTION OF THE INVENTION

### <<Lactic acid bacterium-containing beverage>>

The beverage of the present invention is a lactic acid bacterium-containing beverage characterized by having a pH of 5.0 or higher at 25 °C. The lower limit value (equal to or higher than the value) of the pH at 25°C of the beverage of the present invention is 5.0, preferably 5.5, more preferably 6.0, further preferably 6.5, further more preferably 7.0, and still further more preferably 7.5, from the viewpoint of enhancing the water dispersibility of a lactic acid bacterium in the beverage. On the other hand, the upper limit value (equal to or lower than the value) of the pH at25°C of the beverage of the present invention can be set to, for example, 10.0, 9.5, 9.0, 8.5, or 8.0, from the viewpoint of suitability for beverages. These lower and upper limit values of the pH can be combined arbitrarily. The range of the pH at 25 °C of the beverage of the present invention can be set to, for example, 5.0 to 10.0, 5.5 to 9.5, 6.0 to 9.0, or 6.5 to 8.5. The pH of the beverage can be measured using a commercially available pH meter (for example, a pH meter manufactured by Toa Denpa Kogyo Co. Ltd.).

The beverage of the present invention can comprise a pH-adjusting component. Examples of the pH-adjusting component include citric acid, trisodium citrate, calcium citrate, lactic acid, sodium lactate, calcium lactate, malic acid, sodium malate, carbon dioxide (carbonic acid), potassium carbonate, sodium carbonate, sodium hydrogen carbonate, ammonium bicarbonate, sodium hydroxide, calcium hydroxide, sodium polyphosphate, potassium metaphosphate, sodium metaphosphate, calcium hydrogen phosphate, tripotassium phosphate, tricalcium phosphate, diammonium hydrogen phosphate, dipotassium hydrogen phosphate, disodium hydrogen phosphate, trisodium phosphate, calcium dihydrogen phosphate, sodium dihydrogen phosphate, L-ascorbic acid, and sodium L-ascorbate. While the pH-adjusting component is sometimes referred to as pH-adjusting agent, acidulant, seasoning or the like as a food additive for beverages, any materials will be included in the pH-adjusting component as long as they can adjust the pH of the beverage.

The beverage of the present invention can have a relative turbidity of 0.40 or more. Here, the relative turbidity is defined as a ratio of the turbidity (OD600) of the beverage when 20 hours have passed after start of still-standing (Time: 20 hours (h)) to the turbidity (OD600) of the beverage at the start of the still-standing (Time: 0 hours (h)) in a state where a lactic acid bacterium is dispersed in the beverage, and allowed to stand. The OD600 of the beverage can be measured using a spectrophotometer or micro-volume spectrophotometer, and commercially available apparatuses can be used as the spectrophotometer and micro-volume spectrophotometer. However, in the present invention, a micro-volume spectrophotometer (for example, NanoDrop One^{C} (Thermo Fisher Scientific)) is preferably used for the measurement of OD600, from the viewpoint of high measurement accuracy. In the beverage of the present invention, the relative turbidity is preferably a numerical value calculated based on an average value of the turbidity (OD600) of the beverage obtained through multiple (three) measurements, from the viewpoint of reducing the measurement error, for example, as will be described in the Examples (Example 6) below. The lower limit value (equal to or more than the value) of the relative turbidity of the beverage of the present invention is 0.40, preferably 0.50, more preferably 0.60, further preferably 0.70, further more preferably 0.80, and still further more preferably 0.90, from the viewpoint of enhancing the water dispersibility of the lactic acid bacterium in the beverage. On the other hand, the upper limit value (equal to or less than the value) of the relative turbidity of the beverage of the present invention can be set to, for example, 1.00, 0.99, 0.97, 0.90, 0.80, 0.70 or 0.60, from the viewpoint of suitability for beverages. These lower and upper limit values of the relative turbidity can be combined arbitrarily. The numerical range of the relative turbidity of the beverage of the present invention can be set to, for example, 0.40 to 0.99, 0.50 to 0.97, 0.50 to 0.90, 0.50 to 0.80, or 0.60 to 0.80.

The lactic acid bacterium contained in the beverage of the present invention is not particularly limited, and examples thereof include lactic acid bacteria such as lactic acid cocci belonging to the genus *Lactococcus,* the genus *Leuconostoc,* the genus *Pediococcus,* the genus *Streptococcus,* the genus *Enterococcus,* and the genus *Lactobacillus.*

Examples of the lactic acid cocci belonging to the genus *Lactococcus* include *Lactococcus lactis, Lactococcus lactis* subsp. *lactis, Lactococcus* garvieae, *Lactococcus lactis* subsp. cremoris, and *Lactococcus lactis* subsp. hordniae. *Lactococcus lactis* subsp. *lactis* and *Pediococcus* acidilactici are preferable, *and Lactococcus lactis* subsp. *lactis* is more preferable, from the viewpoint that they are excellent in water dispersibility in the beverage. Specific examples of the bacteria belonging to the *genus Lactococcus* include a *Lactococcus lactis* subsp. *lactis* JCM5805 strain, a *Lactococcus lactis* subsp. *lactis* JCM7638 strain, a *Lactococcus lactis* subsp. *lactis* NRIC1150 strain, and a *Lactococcus lactis* subsp. *lactis* ATCC11454 strain.

Examples of the bacteria belonging to the genus *Leuconostoc* include *Leuconostoc lactis.*

Examples of the bacteria belonging to the genus *Pediococcus* include *Pediococcus* acidilactici, *Pediococcus* pentosaceus, *Pediococcus* cellicola, *Pediococcus* claussenii, *Pediococcus* damnosus, *Pediococcus* ethanolidurans, *Pediococcus* inopinatus, *Pediococcus* parvulus, *and Pediococcus* stilesii. Specific examples of bacteria belonging to the genus *Pediococcus* include a *Pediococcus* acidilactici JCM8797 strain and a *Pediococcus* acidilactici K15 strain. In the present invention, the bacteria belonging to the genus *Pediococcus* can be one or more types selected from the group consisting of: *Pediococcus* cellicola, *Pediococcus* claussenii, *Pediococcus* damnosus, *Pediococcus* ethanolidurans, *Pediococcus* inopinatus, *Pediococcus* parvulus, and *Pediococcus* stilesii, from the viewpoint that they are excellent in water dispersibility in the beverage.

Examples of the bacteria belonging to the genus *Streptococcus* include *Streptococcus thermophilus.*

Examples of the bacteria belonging to the genus *Enterococcus* include *Enterococcus faecalis.*

Examples of the bacteria belonging to the genus *Lactobacillus* include *Lactobacillus paracasei, Lactobacillus delbrueckii, Lactobacillus acidophilus Lactobacillus casei, Lactobacillusfructivorans, Lactobacillus hilgardii, Lactobacillus rhamnosus, Lactobacillus plantarum, Lactobacillus gasseri, and Lactobacillus acidophilus.* Specific examples of the bacteria belonging to the genus *Lactobacillus* include a *Lactobacillus paracasei* KW3110 strain, a *Lactobacillus paracasei* MCC 1849 strain, a *Lactobacillus rhamnosus* GG strain, a *Lactobacillus gasseri* SBT2055 strain, a *Lactobacillus plantarum* L-13 7 strain, and a *Lactobacillus acidophilus* L-92 strain. In the present invention, the bacteria belonging to the genus *Lactobacillus* can be one or more selected from the group consisting of: *Lactobacillus paracasei, Lactobacillus delbrueckii, Lactobacillus acidophilus, Lactobacillus casei, Lactobacillus fructivorans, Lactobacillus hilgardii,* and *Lactobacillus rhamnosus,* from the viewpoint that they are excellent in water dispersibility in the beverage.

In the present invention, the lactic acid bacteria are available from known depositary institutions and the like. For example, among the above lactic acid coccal strains, the JCM bacterial strains are available from Microbe Division, RIKEN BioResource Research Center (3-1-1 Koyadai, Tsukuba, Ibaraki); the NRIC bacterial strains are available from NODAI Culture Collection Center, Tokyo University of Agriculture (1-1-1 Sakuragaoka, Setagaya, Tokyo); and theATCC bacterial strains are available from American Type Culture Collection (U. S.A.). The *Lactobacillus paracasei* KW3110 strain has been deposited, as FERM BP-08634, at International Patent Organism Depositary, National Institute of Advanced Industrial Science and Technology (Central 6, 1-1-1 Higashi, Tsukuba, Ibaraki, 305-8566, Japan) (currently, International Patent Organism Depositary, Biological Resource Center, National Institute of Technology and Evaluation (NITE-IPOD) (#120, 2-5-8 Kazusa-Kamatari, Kisarazu, Chiba, 292-0818), which is the international depositary authority based on the Budapest Treaty for deposit of patent microorganisms (date of deposit: February 20, 2004). In addition, a derivative strain of the *Lactobacillus paracasei* KW3 110 strain has been deposited, asFERMBP-08635, at the same International Patent Organism Depositary.

For the lactic acid bacterium contained in the beverage of the present invention, the median diameter value of lactic acid bacterium particles can be set to 2 µm or less. In the present invention, the lactic acid bacterium particles include aggregates composed of one lactic acid bacterial cell and two or more lactic acid bacterial cells (for example, two lactic acid bacterial cells, three lactic acid bacterial cells, four lactic acid bacterial cells, five lactic acid bacterial cells, or six lactic acid bacterial cells), and the median diameter value of the lactic acid bacterium particles refers to a value measured by a laser diffraction particle size distribution measurement method. The median diameter value of the lactic acid bacterium particles in the beverage of the present invention can be set to preferably 1.8 µm or less and is more preferably 1.6 µm, 1.4 µm or less, or 1.2 µm or less, from the viewpoint of enhancing the water dispersibility of the lactic acid bacterium in the beverage. For the measurement using the laser diffraction particle size measurement method, a commercially available measuring apparatus can be used, such as SALD-2000 series (for example, SALD-2200) manufactured by Shimadzu Corporation. The measurement by the laser diffraction particle size measurement method can be either wet measurement or dry measurement, but wet measurement is preferred from the viewpoint of measuring the median diameter value of the lactic acid bacterium particles of the lactic acid bacterium in the beverage.

The lactic acid bacterium contained in the beverage of the present invention may be present in the form of one lactic acid bacterial cell in the beverage. The beverage of the present invention can have a ratio of one lactic acid bacterial cell to all bacterial cells of 80% or more, preferably 85% or more or 90% or more. The ratio of one lactic acid bacterial cell to all bacterial cells can be measured, for example, in accordance with the method which will be described in the Examples (Example 5) below.

The beverage of the present invention can use a heat-treated lactic acid bacterium bulk powder as a raw material. As the heat-treated lactic acid bacterium bulk powder, the lactic acid bacterium bulk powder of the present invention, which will be described later, can be used from the viewpoint of improving the dispersibility and suppressing the aggregability in the beverage.

In addition to the lactic acid bacterium, a beverage additive that is used in an ordinary beverage formulation design, such as a saccharide, an acidulant, a sweetener, a colorant, a perfume, a seasoning, table salt, an emulsifier, a stabilizer, an amino acid, a vitamin, or a water quality controlling agent may be added, as a raw material, to the beverage of the present invention. The timing for adding these additives is not particularly limited. When a plurality of additives are added, the components may be added together or separately. When they are added separately, any component may be added first. Additionally, those skilled in the art can appropriately select, based on publicly known information, what liquid raw materials or additives should be used depending on the type of beverage to be provided.

The lactic acid bacterium used in the present invention is preferably a dead bacterium from the viewpoint of controlling the number of lactic acid bacteria and maintaining the product quality. As will be described below, a lactic acid bacterium bulk powder obtained by heat treatment (preferably the lactic acid bacterium bulk powder of the present invention obtained by heat treatment at a temperature of 90°C or higher) can also be used.

The beverage of the present invention can be substantially free or completely free of an emulsifier and/or a thickener from the viewpoints of cost, environmental load reduction, and safety. Examples of the case where the beverage is "substantially free of' an emulsifier and/or a thickener include cases where the content of the emulsifier is less than 0.05% by mass (preferably less than 0.03% by mass, more preferably less than 0.01% by mass) relative to the amount of the beverage and cases where the content of the thickener is less than 0.05% by mass (preferably less than 0.03% by mass, more preferably less than 0.01% by mass) relative to the amount of the beverage. The beverage of the present invention can be, for example, a beverage having an emulsifier content of less than 0.05% by mass relative to the amount of the beverage and a thickener content of less than 0.05% by mass relative to the amount of the beverage. The beverage of the present invention is advantageous in that the water dispersibility of the lactic acid bacterium in the beverage can be improved even though the beverage is substantially free of an emulsifier and/or a thickener.

The beverage of the present invention can be substantially free or completely free of a sucrose fatty acid ester (e.g., sucrose stearic acid ester, sucrose oleic acid ester, sucrose lauric acid ester, sucrose palmitic acid ester, or sucrose myristic acid ester) and a polyglycerin fatty acid ester (e.g., polyglycerin fatty acid ester in which a poly glycerin having a polymerization degree of 3 to 10 and a fatty acid having 10 to 18 carbon atoms are ester-bonded). Examples of the case where the beverage is "substantially free of' a sucrose fatty acid ester or a polyglycerin fatty acid ester include cases where the content of the sucrose fatty acid ester is less than 0.05% by mass (preferably less than 0.03% by mass, more preferably less than 0.01% by mass) relative to the amount of the beverage and cases where the content of the polyglycerin fatty acid ester is less than 0.05% by mass (preferably less than 0.03% by mass, more preferably less than 0.01% by mass) relative to the amount of the beverage. The beverage of the present invention can be, for example, a beverage having a sucrose fatty acid ester content of less than 0.05% by mass relative to the amount of the beverage and a polyglycerin fatty acid ester content of less than 0.05% by mass relative to the amount of the beverage. The beverage of the present invention is advantageous in that the water dispersibility of the lactic acid bacterium in the beverage can be improved even though the beverage is substantially free of a sucrose fatty acid ester and/or a polyglycerin fatty acid ester.

The lower limit value (equal to or more than the value) of the number of lactic acid bacterium contained in the beverage of the present invention can be set to 1 million/500 mL, 10 million/500 mL, 100 million/500 mL, 1 billion/500 mL, or 10 billion/500 mL, and the upper limit value (equal to or less than the value) thereof can be set to 100 trillion/500 mL, 10 trillion/500 mL, or 1 trillion/500 mL. These lower and upper limit values of the number of bacteria can be combined arbitrarily. The number of lactic acid bacterium in the beverage of the present invention can be set to 1 million/500 mLto 100 trillion/500 mL, preferably 100 million/500 mLto 10 trillion/500 mL, more preferably 1 billion/500 mL to 1 trillion/500 mL, and further preferably 10 billion/500 mL to 1 trillion/500 mL. The number of lactic acid bacterium can be measured using a microscope, a flow cytometer, a non-cultured microorganism rapid testing device (e.g., ELESTAPixeeMo (AFI Technology)) or the like, and the number of bacterium in the beverage can be adjusted as necessary. The measurement using a microscope is preferred from the viewpoint of high versatility.

The beverage of the present invention is not particularly limited as long as it is allowed to contain a lactic acid bacterium, and can be provided as a beverage that does not contain an alcohol (non-alcoholic beverage) or a beverage that does not use barley or malt as a raw material (non-barley beverage), and examples thereof include flavored water drinks, sports drinks (isotonic drinks), mineral water, fruit juice drinks, milk drinks, vegetable juice drinks, acidic drinks, carbonated drinks, whey drinks, coffee drinks, black tea drinks, green tea drinks, oolong tea drinks, barley tea drinks, and energy drinks.

The beverage of the present invention can be provided in a packaged form. The container in which the beverage is packaged is not particularly limited as long as it can be provided as a beverage container such as a bottle, a can, ajar or the like, and the material for the container can be appropriately selected from materials suitable for the beverage container, such as glass and plastic (including biodegradable plastic). That is, specific examples of the container for the beverage of the present invention include glass jars and plastic bottles.

### <<Method for producing lactic acid bacterium-containing beverage>>

The beverage of the present invention can be produced by carrying out the steps of: (A) adjusting the pH of the beverage at 25 °C to 5.0 or higher, and/or (B) blending a lactic acid bacterium bulk powder obtained by heat-treating a lactic acid bacterium at a temperature of 90°C or higher.

In the step (A), the pH of the beverage at25°C is adjusted to 5.0 or higher. The pH can be adjusted by including a pH-adjusting component into the beverage. Usable pH-adjusting components are those which are described in the context of the beverage of the present invention. Also, the pH of the beverage can be measured according to the descriptions regarding the beverage of the present invention. The production method of the present invention may comprise the step of (C) blending a lactic acid bacterium bulk powder into the beverage or raw materials for the beverage before or after the step (A), or simultaneously with the step (A). The lactic acid bacterium can be blended according to a conventional method.

In the step (B), a lactic acid bacterium bulk powder obtained by heat-treating a lactic acid bacterium at a temperature of 90°C or higher is blended into the beverage or raw materials for the beverage. Usable lactic acid bacterium bulk powders are those which will be described as the lactic acid bacterium bulk powder of the present invention later, or lactic acid bacterium bulk powders obtained by the production method which will be described as the method for producing a lactic acid bacterium bulk powder of the present invention later. The lactic acid bacterium bulk powder used in the step (B) is obtained by heat treatment. The method for heat-treating the lactic acid bacterium bulk powder can comprise, for example, the step of (D) heat-treating a bacterial solution containing the lactic acid bacterium at a temperature of 90°C or higher, for example, before the step (B), but is not limited thereto.

In the production method of the present invention, the step (A) and the step (B) may be carried out alone or in combination. When the step (A) and the step (B) are carried out in combination in the production method of the present invention, the step (B) may be carried out before or after the step (A), or simultaneously with the step (A).

The method for producing a beverage according to the present invention can further comprise the step of (E) measuring the number of lactic acid bacterial cells in the lactic acid bacterium-containing beverage. The number of lactic acid bacterial cells can be measured using a microscope, a flow cytometer, a non-cultured microorganism rapid testing device (e.g., ELESTAPixeeMo (AFI Technology)) or the like, as described above, and the measurement using a microscope is preferred from the viewpoint of high versatility.

### <<Lactic acid bacterium bulk powder>>

The lactic acid bacterium bulk powder of the present invention is obtained by heat-treating a lactic acid bacterium at a temperature of 90°C or higher. The heat treatment can be performed by heating a bacterial solution containing the lactic acid bacterium. The lactic acid bacterium bulk powder of the present invention may be composed of a dead bacterium since the lactic acid bacterium in the lactic acid bacterium bulk powder can be killed by the heat treatment. Here, the bacterial solution includes, for example, a lactic acid bacterium-containing culture solution (the same applies hereinafter in the present specification). The heat treatment can be performed by a conventional method, and, for example, can be carried out by heat-treating a lactic acid bacterium-containing bacterial solution using a heat treatment apparatus such as a plate-type sterilizer, a tubular-type sterilizer, a direct heating sterilizer, a jacketed tank or the like.

The lower limit value (equal to or higher than the value) of the temperature for the heat treatment is 90°C, preferably 95°C, more preferably 100°C, and further preferably 105 °C from the viewpoint of enhancing the water dispersibility of the lactic acid bacterium in the beverage, and the upper limit value (equal to or lower than the value) is 120°C, preferably 110°C since a maximally low total heat history for the lactic acid bacterium is desirable and from the viewpoint of cost. These upper and lower limit values of the heating temperature can be combined arbitrarily, and the range of the heating temperature can be set to, for example, 90 to 120°C or 95 to 110°C, and preferably 100 to 110°C in consideration of enhancing the water dispersibility of the lactic acid bacterium, and the balance with the total heat history for the lactic acid bacterium and the cost.

The time for the heat treatment can be set to 30 to 90 minutes (preferably 30 to 60 minutes) from similar viewpoints as those for the temperature for the heat treatment, and can also be the time necessary to kill the lactic acid bacterium.

The lactic acid bacterium bulk powder of the present invention can have a ratio of one lactic acid bacterial cell to all bacterial cells of 80% or more. The ratio of one lactic acid bacterial cell to all bacterial cells is preferably 85% or more, more preferably 90% or more, further preferably 95% or more, further more preferably 98% or more, and still further more preferably 99% or more, from the viewpoint of enhancing the water dispersibility of the lactic acid bacterium in the beverage.

The lactic acid bacterium bulk powder of the present invention is a composition containing one lactic acid bacterial cell and/or two or more lactic acid bacterial cells as the number of bacterial cells. When the lactic acid bacterium bulk powder of the present invention is contained in the beverage, the range of the number of bacterial cells in the lactic acid bacterium particles in the beverage is one to six bacterial cells, preferably one to five bacterial cells, more preferably one to four bacterial cells, further preferably one to three bacterial cells, further more preferably one to two bacterial cells, and still further more preferably one bacterial cell, from the viewpoint of enhancing the water dispersibility of the lactic acid bacterium in the beverage.

In the lactic acid bacterium bulk powder of the present invention, the adhesive force of lactic acid bacterial cells in the lactic acid bacterium bulk powder can be set to 0.83 or less, and can be preferably 0.57 or more and 0.83 or less, relative to the adhesive force of lactic acid bacterial cells in the lactic acid bacterium bulk powder heat-treated at 80°C. The adhesive force of lactic acid bacterial cells can be evaluated, for example, according to the descriptions in the Examples (Example 4) below. It is possible to prepare force curves when a probe is pushed onto the lactic acid bacterium and when the probe is retracted therefrom, using an atomic force microscope (AFM), and to determine the adhesive force (nN) of the lactic acid bacterial cells from the maximum value of the force applied to a cantilever at the time of retraction.

### <<Method for producing lactic acid bacterium bulk powder>>

The method for producing a lactic acid bacterium bulk powder according to the present invention can comprise the step of (D) heat-treating a lactic acid bacterium-containing bacterial solution at a temperature of 90 °C or higher. The heat treatment can be performed according to the above descriptions regarding the lactic acid bacterium bulk powder. Also, in the step (D), the lactic acid bacterium can be subjected to the heat treatment in the form of a lactic acid bacterium-containing bacterial solution. In the step (D), the lactic acid bacterium in the bacterial solution can also be killed (sterilized). The method for producing a lactic acid bacterium bulk powder according to the present invention is advantageous in that, since the heat treatment can enhance the water dispersibility of the lactic acid bacterium in the beverage and simultaneously kill the lactic acid bacterium, it is possible to reduce the total heat history for the lactic acid bacterium and to suppress the cost for the heat treatment.

The method for producing a lactic acid bacterium bulk powder according to the present invention can comprise the step of (X) washing the lactic acid bacterium-containing bacterial solution before the step (D). In the step (X), the washing can be performed by mixing the lactic acid bacterium and phosphate buffered saline (PBS) (for example, mixing a lactic acid bacterium-containing culture solution and PBS at an appropriate ratio) and centrifuging the mixture. The number of times of washing the lactic acid bacterium can be one or more (preferably two) from the viewpoint of eliminating a medium contained in the lactic acid bacterium and acquiring a purified lactic acid bacterium bulk powder.

The method for producing a lactic acid bacterium bulk powder of the present invention can comprise the step of (Y) drying the lactic acid bacterium after the step (D). The drying can be performed according to a conventional method such as spray drying or freeze drying.

### <<Method for improving water dispersibility of lactic acid bacterium>>

The method for improving the water dispersibility of a lactic acid bacterium in a lactic acid bacterium-containing beverage according to the present invention is characterized by comprising the steps of: (F) adjusting the pH of the beverage at 25 °C to 5.0 or higher, and/or (G) blending a lactic acid bacterium bulk powder obtained by heat-treating a lactic acid bacterium at a temperature of 90°C or higher. The step (F) correspondsto the step (A) and can be carried out according to the descriptions regarding the lactic acid bacterium-containing beverage of the present invention and the method for producing the same. The step (G) corresponds to the step (B) and can be carried out according to the descriptions regarding the lactic acid bacterium bulk powder of the present invention and the method for producing the same. The method for improving the water dispersibility of a lactic acid bacterium according to the present invention is advantageous in that, since the method can enhance the water dispersibility of a lactic acid bacterium in a beverage, the number of lactic acid bacterial cells contained in the beverage can be measured conveniently and efficiently. Especially, the method is advantageous in that the number of lactic acid bacterial cells contained in the beverage can be measured conveniently and efficiently without addition of an emulsifier and/or a thickener to the beverage.

### EXAMPLES

Hereinafter, the present invention will be described in more detail by way of the following examples, but is not limited thereto.

### Example 1: Study (1) on improvement of water dispersibility of lactic acid bacterium bulk powder

In Example 1, the influence of the pH on the water dispersibility of a lactic acid bacterium was studied.

### (1) Method

### A. Bacterial strain

*A Lactococcus lactis* subsp. *lactis* JCM5805 strain was used as the lactic acid bacterium.

### B. Preparation of lactic acid bacterium bulk powder

A lactic acid bacterium bulk powder was prepared by the following procedures (i) to (iv).

### (i) Culture conditions

The used medium was MRS medium (MRS BROTH, CODE: CM0359, Oxoid; the composition thereof is indicated in Table 1). The lactic acid bacterium was inoculated (seeded) in a 15-ml conical tube (FALCON) so that the concentration of the lactic acid bacterium reached 0.1% (v/v) per 10 ml of the prepared medium, and cultured at 30°C for 24 hours in an incubator (TOKYO RIKAKIKAI CO., LTD.) (static culture).

**[Table 1]**

| Table 1: Composition of MRS medium | |
|---|---|
| | g/L |
| Peptone | 10.0 |
| Lab-Lemco powder (beef extract) | 8.0 |
| Yeast extract | 4.0 |
| Glucose | 20.0 |
| Sorbitan oleate | 1 ml |
| Dipotassium hydrogen phosphate | 2.0 |
| Sodium acetate trihydrate | 5.0 |
| Triammonium citrate | 2.0 |
| Magnesium sulfate heptahydrate | 0.2 |
| Manganese sulfate tetrahydrate | 0.05 |
| Purified water | Balance |

### (ii) Centrifugation (acquisition of concentrated bacterial solution) and washing

The culture solution obtained in the above (i) was concentrated by centrifugation (5000 rpm), and the supernatant was removed, thereby obtaining a bacterial solution. Subsequently, the obtained bacterial solution and phosphate buffered saline (PBS) (Takara Bio Inc.) were mixed at a ratio of 1:19, and washed twice by centrifugation (5000 rpm).

### (iii) Heat treatment

The bacterial solution washed in the above (ii) was heat-treated to kill the lactic acid bacterium in the bacterial solution. During the heat treatment, the temperature was increased from room temperature to 90°C over 30 minutes, retained at 90°C for 30 minutes, and then decreased to room temperature over 30 minutes.

### (iv) Spray drying (spray dryer)

The bacterial solution heat-treated in the above (iii) was spray-dried to obtain a lactic acid bacterium bulk powder (lactic acid bacterium powder).

### C. Preparation of solution

The lactic acid bacterium bulk powder obtained in the above B was dispersed in purified water by stirring so as to attain 0.1 mg/ml. Next, trisodium citrate and phosphoric acid were added to this solution to adjust the pH to 3.8, and the solution was then heated at 65°C for 10 minutes to obtain the desired solution (Test Plot 1). In addition, sodium hydroxide was added to the solution of Test Plot 1 to prepare solutions whose pH values were adjusted to 5.0 to 10.0 (Test Plots 2 to 6).

### D. Measurement of particle size distribution

The lactic acid bacterium was collected from each of the solutions prepared in the above C by centrifugation (5000 rpm) and dispersed in PBS solutions whose pH values were adjusted to 3.8 to 10.0 by stirring. Thereafter, 10 ml of the dispersion liquid that was sonicated (oscillation frequency: 28 kHz, power: 4 W) using an ultrasonic generator (UR-21P, TOMY SEIKO CO., LTD.) was labeled as "with sonication," and 10 ml of the dispersion liquid that was subjected to maximum-strength vortex (Electro Scientific Industries) for 10 seconds without sonication was labeled as "without sonication." These PBS solutions were then subjected to SALD-2200 (Shimadzu Corporation), and particle size distributions were measured by the laser diffraction particle size distribution measuring method (wet).

### (2) Result

The results were as shown in FIG. 1. It was confirmed that, in Test Plots 2 to 6 (pH: 5.0 to 10.0), both the median diameter value and the average value, for the particle size of the lactic acid bacterium particles, were small in both "with sonication" and "without sonication," as compared with those in Test Plot 1 (pH: 3.8). These results showed that the dispersibility of the lactic acid bacterium improved when the pH of the aqueous solution was 5.0 or higher.

### Example 2: Study (2) on improvement of water dispersibility of lactic acid bacterium bulk powder

In Example 2, the influence of the temperature for heat treatment in the process for preparing a lactic acid bacterium bulk powder on the water dispersibility of a lactic acid bacterium was studied.

### (1) Method

### A. Bacterial strain

The same bacterial strain as that described in Example 1, item (1), A was used.

### B. Preparation of lactic acid bacterium bulk powder

A lactic acid bacterium bulk powder was prepared by the following procedures (i) to (iv).

### (i) Culture conditions

Culture was performed under the same culture conditions as described in Example 1, item (1), B, (i).

### (ii) Centrifugation (acquisition of concentrated bacterial solution) and washing

Centrifugation and washing were performed in the same manner as described in Example 1, item (1), B, (i).

### (iii) Heat treatment

Heat treatment was performed in the same manner as described in Example 1, item (1), B, (iii), except that the temperature for heat treatment was set to four temperatures, 80°C, 90°C, 95°C, and 105°C, to kill the lactic acid bacterium.

### (iv) Spray drying (spray dryer)

Spray drying was performed in the same manner as described in Example 1, item (1), B, (iv).

### C. Preparation of solution

Solutions were prepared in the same manner as described in Example 1, item (1), C, except that the pH values of the solutions were adjusted to 7.5.

### D. Measurement of particle size distribution

The lactic acid bacterium was collected from each of the solutions prepared in the above C by centrifugation (5000 rpm) and dispersed in PBS solutions each having a pH adjusted to 7.5. Then, 10 ml of the dispersion liquid was sonicated (oscillation frequency: 28 kHz, power: 4 W) using an ultrasonic generator (UR-21P, TOMY SEIKO CO., LTD.). The PBS solution was then subjected to SALD-2200 (Shimadzu Corporation), and particle size distributions were measured 4 hours after the sonication and 24 hours after the sonication.

### (2) Result

The results were as shown in FIG. 2. It was confirmed that, in Test Plots 8 to 10 (90 to 105 °C), both the median diameter value and the average value, for the particle size of the lactic acid bacterium particles, were small both 4 hours and 24 hours after the sonication, as compared with those in Test Plot 7 (80°C). These results showed that the dispersibility of the lactic acid bacterium improved (the lactic acid bacterium became less likely to aggregate) when the temperature for heat treatment in the process for preparing a lactic acid bacterium bulk powder was 90°C or higher, and that the retention of the dispersibility of the lactic acid bacterium was improved (the lactic acid bacterium became less likely to reaggregate). The dispersibility of a lactic acid bacterium refers to the degree of dispersibility when a lactic acid bacterium bulk powder is mixed (dispersed) in water, and the retention of the dispersibility of a lactic acid bacterium refers to the degree of dispersibility when a certain period of time has passed after the lactic acid bacterium bulk powder is mixed (dispersed) in water.

### Example 3: Study (3) on improvement of water dispersibility of lactic acid bacterium bulk powder

In Example 3, the influence of the pH on the water dispersibility of a lactic acid bacterium was studied using the lactic acid bacterium bulk powder heat-treated at 105°C in Example 2.

### (1) Method

### A. Bacterial strain

The same bacterial strain as that described in Example 1, item (1), A was used.

### B. Preparation of lactic acid bacterium bulk powder

A lactic acid bacterium bulk powder was prepared by the following procedures (i) to (iv).

### (i) Culture condition

Culture was performed under the same culture conditions as described in Example 1, item (1), B, (i).

### (ii) Centrifugation (acquisition of concentrated bacterial solution) and washing

Centrifugation and washing were performed in the same manner as described in Example 1, item (1), B, (i).

### (iii) Heat treatment

Heat treatment was performed in the same manner as described in Example 1, item (1), B, (iii), except that the temperature for heat treatment was set to 105°C, to kill the lactic acid bacterium.

### (iv) Spray drying (spray dryer)

Spray drying was performed in the same manner as described in Example 1, item (1), B, (iv).

### C. Preparation of solution

Solutions were prepared in the same manner as described in Example 1, item (1), C, except that the pH values of the solutions were adjusted to four values, i.e., 3.8, 7.5, 9.0 and 10.0.

### D. Measurement of particle size distribution

Particle size distributions were measured in the same manner as described in Example 1, item (1), D, except that the lactic acid bacterium was dispersed in PBS solutions whose pH values were adjusted to four values, i.e., 3.8, 7.5, 9.0 and 10.0.

### (2) Result

The results were as shown in FIG. 3. It was confirmed that, in Test Plots 12 to 14 (pH: 7.5 to 10.0), both the median diameter value and the average value, for the particle size of the lactic acid bacterium particles, were small in both "with sonication" and "without sonication," as compared with those in Test Plot 11 (pH: 3.8). These results showed that, also when the temperature for heat treatment in the process for preparing a lactic acid bacterium bulk powder was 105°C, the dispersibility of the lactic acid bacterium and the retention of the dispersibility of the lactic acid bacterium were further improved by setting the pH to 7.5 or higher. Therefore, it was shown that the dispersibility of the lactic acid bacterium was synergistically improved by setting the heating temperature and the pH of the solution within the predetermined numerical ranges.

### Example 4: Evaluation of adhesive force of lactic acid bacterial cell in lactic acid bacterium bulk powder

In Example 4, the influence of the temperature for heat treatment in the process for preparing a lactic acid bacterium bulk powder on the adhesive force of lactic acid bacterial cells was studied.

### (1) Method

### A. Lactic acid bacterium bulk powder

Among the lactic acid bacterium bulk powders in Example 2, the lactic acid bacterium bulk powders heat-treated at 80°C and 105°C in Example 2, item (1), B were used.

### B. Evaluation of adhesive force of lactic acid bacterial cell

To a slide glass, 10 µL of a 0.1 w/v% aqueous poly-L-lysine solution (Sigma Aldrich, Product No. P8920-100ML) was added, and incubated for 5 minutes at room temperature. Then, the glass was washed with pure water. To this slide glass, 10 µL of a bacterial cell suspension prepared by adding pure water to each of the above-described lactic acid bacterium bulk powders was applied, and allowed to stand for 30 minutes. Thereafter, an excessive bacterium was sucked up. Water was then dropped upon observation, and atomic force microscopic (AFM) observation in water was performed. NanoWizard4 (Bruker) was used as the AFM, and SNL-10B (resonance frequency: 23 kHz, spring constant: 0.12 N/m, tip diameter: 2 nm, Bruker) was used as a probe. The observation was performed in the Quantitative Imaging mode, force curves when the probe was pushed onto the sample and when the probe was retracted therefrom were prepared, and the maximum value of the force applied to a cantilever at the time of retraction was determined, thereby deriving the adsorption force.

### (2) Result

The results were as shown in FIGS. 4 and 5. It was confirmed that the adhesive force of the lactic acid bacterial cells in the lactic acid bacterium bulk powder heat-treated at 105 °C was reduced as compared with that of the lactic acid bacterial cells in the lactic acid bacterium bulk powder heat-treated at 80°C (FIG. 4). Also, from a result of the AFM observation of the unevenness of the surface of the bacterial cell, it was confirmed that the unevenness of the surface of the lactic acid bacterial cell in the lactic acid bacterium bulk powder heat-treated at 105°C (FIG. 5B) was increased as compared with that of the surface of the lactic acid bacterial cell in the lactic acid bacterium bulk powder heat-treated at 80°C (FIG. 5A). Specifically, in an enlarged image of the surface of the lactic acid bacterial cell in the lactic acid bacterium bulk powder heat-treated at 80°C, the surface of the lactic acid bacterial cell was confirmed to be flat in color tone (less uneven) (FIG. 5A, right figure (enlarged image of a portion put in a square in FIG. 5A, left figure). On the other hand, in an enlarged image of the surface of the lactic acid bacterial cell in the lactic acid bacterium bulk powder heat-treated at 105°C, the surface of the lactic acid bacterial cell was confirmed to be variable in color tone (more uneven) (FIG. 5B, lower figure (enlarged image of a portion put in a square in FIG. 5B, upper figure). From these results, it was considered that, at a high temperature for heat treatment, the adhesive force of the lactic acid bacterial cells decreased due to the increase in unevenness of the surface of the bacterial cells.

### Example 5: Evaluation of dispersibility of lactic acid bacterium in aqueous solution

In Example 5, the influence of the temperature for heat treatment in the process for preparing a lactic acid bacterium bulk powder on the aggregability of the lactic acid bacterium was evaluated.

### (1) Method

### A. Lactic acid bacterium bulk powder

Among the lactic acid bacterium bulk powders in Example 2, the lactic acid bacterium bulk powders heat-treated at 80°C and 105°C in Example 2, item (1), B were used.

### B. Evaluation of aggregability of lactic acid bacterium

Five (5) µL of a bacterial cell suspension prepared by adding pure water to each of the above-described lactic acid bacterium bulk powders was dropped onto a slide glass, which was cover with a cover glass (18 mm × 18 mm), and sealed with a top coat to make a preparation. It was allowed to stand for about 30 minutes to cause the bacterium to be attached to the observation surface gently. Thereafter, it was observed by differential interferometry using a confocal laser microscope (FV1000, Olympus). The bacterial suspension was observed using a 100× objective lens, and set to a concentration at which approximately 5 to 20 bacteria could be observed per 64 µm-square field of view. It was observed how many bacterial cells were contained in one particle to calculate the total number of particles in 20 random fields of view, the number of bacterial cells contained per particle, and the total number of bacterial cells.

### (2) Result

The results were as shown in FIG. 6. It was confirmed that the aqueous solutions using the lactic acid bacterium bulk powder heat-treated at 105°C had a high proportion of lactic acid bacteria which were present in the state of one bacterial cell and a low proportion of lactic acid bacteria which were present in the state of two bacterial cells, as compared with the aqueous solutions using the lactic acid bacterium bulk powder heat-treated at 80°C. From these results, it was seen that, at a high temperature for heat treatment, the adhesive force of the lactic acid bacterial cells decreased due to the increase in unevenness of the surface of the bacterial cells from the morphological viewpoint, resulting in deteriorated aggregability.

### Example 6: Study (4) on improvement of water dispersibility of lactic acid bacterium bulk powder

In Example 6, the influence of the pH and temperature for heat treatment in the process for preparing a lactic acid bacteria bulk powder on the water dispersibility of a lactic acid bacterium was further studied.

### (1) Method

### A. Bacterial strain

The same bacterial strain as that described in Example 1, item (1), A was used.

### B. Preparation of lactic acid bacterium bulk powder

A lactic acid bacterium bulk powder was prepared by the following procedures (i) to (iv).

### (i) Culture condition

Culture was performed under the same culture conditions as described in Example 1, item (1), B, (i).

### (ii) Centrifugation (acquisition of concentrated bacterial solution) and washing

Centrifugation and washing were performed in the same manner as described in Example 1, item (1), B, (i).

### (iii) Heat treatment

Heat treatment was performed in the same manner as described in Example 1, item (1), B, (iii), except that the temperature for heat treatment was set to 80°C or 105°C, to kill the lactic acid bacterium.

### (iv) Spray drying (spray dryer)

Spray drying was performed in the same manner as described in Example 1, item (1), B, (iv).

### C. Measurement of turbidity OD600

The lactic acid bacterium bulk powder obtained in the above B was dispersed in PBS (10 ml) whose pH was adjusted to 3.8, 7.0 or 10.0 by stirring so as to attain 0.1 mg/ml. Next, 1.0 ml of each PBS was dispensed into a 1.5 -ml microtube (Eppendorf), and vortex (Electro Scientific Industries) was performed at the maximum intensity for 10 seconds. Then, the PBS (1 µl) at a position 1.0 cm distant from the gas-liquid interface was subjected to a next-generation micro-volume spectrophotometer NanoDrop One^{C} (Thermo Fisher Scientific) to measure OD600 (Time: 0) at start of still-standing (Time: 0 h) three times and to calculate an average value thereof. Next, when 20 hours had passed after the start of the still-standing (Time: 20 h), OD600 (Time: 20 h) was similarly measured three times and an average value thereof was calculated. Then, for each sample, the relative turbidity was calculated by dividing OD600 (Time: 20 h) by OD600 (Time: 0 h) based on these average values. Here, the state of a low relative turbidity is a state where aggregation of lactic acid bacteria and sedimentation of lactic acid bacteria are promoted (low water dispersibility of lactic acid bacteria), and the state of a high relative turbidity is a state where aggregation of lactic acid bacteria and sedimentation of lactic acid bacteria are not promoted (high water dispersibility of lactic acid bacteria).

### (2) Result

The results were as shown in FIGS. 7 to 9. Specifically, it was confirmed that the relative turbidity increased regardless of whether the temperature for heat treatment was set to 80°C or 105°C (FIGS. 7 and 8). The results of the Tukey test showed that, when the temperature for heat treatment was set to 80°C, the test plots of a pH of 7.0 and a pH of 10.0 exhibited a significant difference in increase in relative turbidity as compared with the test plot of a pH of 3 .8, and that a significant difference in increase in relative turbidity was found in the test plot of a pH of 10.0 as compared with the test plot of a pH of 7.0 (FIG. 7). It was also confirmed that the relative turbidity increased in all of the other five test plots as compared with the test plot of a temperature for heat treatment of 80°C and a pH of 3.8, and the results of the Dunnett test showed that there was a significant difference in this increase (FIG. 9). These results showed that the aggregation of lactic acid bacteria when the solutions were allowed to stand was suppressed by increasing the pH.

### Example 7: Study (5) on improvement of water dispersibility of lactic acid bacterium bulk powder

In Example 7, the influence of the temperature for heat treatment in the process for preparing a lactic acid bacterium bulk powder on the water dispersibility of a lactic acid bacterium was studied using bacterial strains other than the lactic acid bacterial strain used in Example 1.

### (1) Method

### A. Bacterial strain

The lactic acid bacterial strains indicated in Table 2 were used as lactic acid bacteria.

**[Table 2]**

| Table 2: List of lactic acid bacterial strains | |
|---|---|
| 1. ATCC11454 | *Lactococcus lactis* subsp. *lactis* ATCC11454 strain |
| 2. JCM763 8 | *Lactococcus lactis* subsp. *lactis* JCM763 8 strain |
| 3. JCM8797 | *Pediococcus acidilactici* JCM8797 strain |
| 4. NRIC1150 | *Lactococcus lactis* subsp. *lactis* NRIC1150 strain |
| 5. KW3110 | *Lactobacillus paracasei* KW3110 strain |

### B. Preparation of lactic acid bacterium bulk powder

A lactic acid bacterium bulk powder was prepared by the following procedures (i) to (iv).

### (i) Culture conditions

Culture was performed under the same culture conditions as described in Example 1, item (1), B, (i).

### (ii) Centrifugation (acquisition of concentrated bacterial solution) and washing

Centrifugation and washing were performed in the same manner as described in Example 1, item (1), B, (i).

### (iii) Heat treatment

Heat treatment was performed in the same manner as described in Example 1, item (1), B, (iii), except that the temperature for heat treatment was set to four temperatures, i.e., 80°C, 90°C, 95°C, and 105°C, to kill the lactic acid bacterium.

### (iv) Spray drying (spray dryer)

Spray drying was performed in the same manner as described in Example 1, item (1), B, (iv).

### C. Preparation of solution

A solution was prepared by dispersing the lactic acid bacterium bulk powder obtained in the above B in PBS whose pH was adjusted to 3.8, so as to reach a concentration of 0.1 mg/ml.

### D. Measurement of particle size distribution

Each of the solutions prepared in the above C was sonicated (oscillation frequency: 28 kHz, power: 4 W) using an ultrasonic generator (UR-21P, TOMY SEIKO CO., LTD.). The PBS solution was then subjected to SALD-2200 (Shimadzu Corporation), and a particle size distribution was measured by the laser diffraction particle size distribution measuring method (wet).

### (2) Result

The results were as shown in FIG. 10. For the lactic acid bacteria studied, it was confirmed that, when the temperature for heat treatment was increased from 80°C to 105°C, both the median diameter value and the average value, for the particle size of the lactic acid bacterium particles, were decreased. These results showed that, when the temperature for heat treatment was increased from 80°C to 105°C in the process for preparing the bulk lactic acid bacterium powder containing a plurality of types of lactic acid bacteria belonging to *Lactococcus* lactis subsp. lactis and *Pediococcus* and *Pediococcus* acidilactici, the dispersibility of the lactic acid bacteria was improved (the lactic acid bacteria became less likely to aggregate).

## Claims

1. A lactic acid bacterium-containing beverage having a pH of 5.0 or higher at 25°C.

2. The beverage according to claim 1, which has a relative turbidity of 0.40 or more, wherein the "relative turbidity" is defined as a ratio of the turbidity (OD600) of the beverage when 20 hours have passed after start of still-standing to the turbidity (OD600) of the beverage at the start of the still-standing (0 hours) in a state where a lactic acid bacterium is dispersed in the beverage, and allowed to stand.

3. The beverage according to claim 1 or 2, wherein the median diameter value of lactic acid bacterium particles, as measured by a laser diffraction particle size distribution measurement method, is 2 µm or less.

4. The beverage according to claim 1 or 2, wherein a heat-treated lactic acid bacterium bulk powder is used as a raw material.

5. The beverage according to claim 1 or 2, wherein the lactic acid bacterium is a dead bacterium.

6. The beverage according to claim 1 or 2, which is substantially free of an emulsifier and/or a thickener.

7. The beverage according to claim 1 or 2, wherein the number of lactic acid bacteria is 1 million or more/500 mL.

8. The beverage according to claim 1 or 2, which is a packaged beverage.

9. The beverage according to claim 1 or 2, wherein the lactic acid bacterium is a lactic acid coccus.

10. The beverage according to claim 9, wherein the lactic acid coccus is a bacterium belonging to the genus *Lactococcus.*

11. The beverage according to claim 10, wherein the bacterium belonging to the genus *Lactococcus* is a *Lactococcus lactis* subsp. *lactis* JCM5805 strain.

12. A method for producing a lactic acid bacterium-containing beverage, comprising the steps of: (A) adjusting the pH of the beverage at 25 °C to 5.0 or higher, and/or (B) blending a lactic acid bacterium bulk powder obtained by heat-treating a lactic acid bacterium at a temperature of 90°C or higher.

13. The method for producing a lactic acid bacterium-containing beverage according to claim 12, further comprising the step of (E) measuring the number of lactic acid bacterial cells in the lactic acid bacterium-containingbeverage.

14. A lactic acid bacterium bulk powder obtained by heat treatment at a temperature of 90°C or higher.

15. The lactic acid bacterium bulk powder according to claim 14, wherein the ratio of one lactic acid bacterial cell to all bacterial cells is 80% or more.

16. The lactic acid bacterium bulk powder according to claim 13 or 14, wherein the adhesive force of lactic acid bacterial cells in the lactic acid bacterium bulk powder is 0.83 or less relative to the adhesive force of lactic acid bacterial cells in the lactic acid bacterium bulk powder heat-treated at 80°C.

17. A method for producing a lactic acid bacterium bulk powder, comprising the step of (D) heat-treating a lactic acid bacterium at a temperature of 90 °C or higher.

18. The method for producing a lactic acid bacterium bulk powder according to claim 17, wherein the lactic acid bacterium is killed in the step (D).

19. A lactic acid bacterium bulk powder produced by the method for producing a lactic acid bacterium bulk powder according to claim 17 or 18.

20. A method for improving the water dispersibility of a lactic acid bacterium in a lactic acid bacterium-containing beverage, comprising the steps of: (F) adjusting the pH of the beverage at 25 °C to 5.0 or higher, and/or (G) blending a lactic acid bacterium bulk powder obtained by heat-treating a lactic acid bacterium at a temperature of 90°C or higher.

21. The method for improving the water dispersibility of a lactic acid bacterium according to claim 20, for use in measuring the number of lactic acid bacterial cells contained in the lactic acid bacterium-containing beverage.
